Europäisches Patentamt

**European Patent Office**

**Office européen des brevets**

(19)

(11) Publication number: **0 118 286 B1**

# EUROPEAN PATENT SPECIFICATION

(12)

(45) Date of publication of patent specification: **17.04.91**   (51) Int. Cl.⁵: **A61K 35/00, A61K 35/23**

(21) Application number: **84301350.9**

(22) Date of filing: **01.03.84**

The file contains technical information submitted after the application was filed and not included in this specification

(54) **Process for preparing an inhibiting agent.**

<table>
<tr><td>

(30) Priority: **07.03.83 JP 35850/83**

(43) Date of publication of application:
**12.09.84 Bulletin 84/37**

(45) Publication of the grant of the patent:
**17.04.91 Bulletin 91/16**

(84) Designated Contracting States:
**CH DE FR GB LI NL**

(56) References cited:
**EP-A- 0 087 087**   **EP-A- 0 102 801**
**EP-A- 0 125 125**   **EP-A- 0 133 029**
**EP-A- 0 133 358**   **EP-A- 0 136 053**

**BIOLOGICAL ABSTRACTS, vol. 66, 1978, no. 41061, Phila., Pa, US; D.L. DEWEY: "The identification of a cell culture inhibitor in a tumour extract", & CANCER LETT. 4(2): 77-84, 1978**

</td><td>

(73) Proprietor: **KOKEN LTD.**
**No. 7, Yonban-cho**
**Chiyoda-ku Tokyo(JP)**

Proprietor: **Nagasu, Youichiro**
**No. 9-4, Chuo 3-chome**
**Yamato-shi Kanagawa-ken(JP)**

Proprietor: **Tajima, Tomoyuki**
**5-15, Yawata 6-chome**
**Ichikawa-shi Chiba-ken(JP)**

(72) Inventor: **Tajima, Tomoyuki**
**No. 5-15, Yawata 6-chome**
**Ichikawa-shi Chiba-ken(JP)**

(74) Representative: **Deans, Michael John Percy et al**
**Lloyd Wise, Tregear & CO. Norman House**
**105-109 Strand**
**London WC2R OAE(GB)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

BIOLOGICAL ABSTRACTS, vol. 65, 1978, no. 3219, Phila., Pa, US; D.M. BURNS et al.: "Natural occurrence of an inhibitor of mammalian cell growth in human and mouse cells of normal and tumor origin", & CANCER BIOCHEM. BIOPHYS. 1(6): 269-280 1976

BIOLOGICAL ABSTRACTS, vol. 65, 1978, no. 34666, Phila., Pa, US; G.L. WRIGHT, Jr. et al.: "Isolation of a soluble tumor-associated antigen from human renal cell carcinoma by gradient acrylamide gel electrophoresis", & CANCER RES. 37(11): 4228-4232, 1977

BIOLOGICAL ABSTRACTS, vol. 65, 1978, no. 16073, Phila., Pa., US; J.L. MCCOY et al.: "Leukocyte migration inhibition in patients with Ewing's sarcoma by 3-M potassium chloride extracts of fresh and tissue-cultured Ewing's sarcomas", & J. NATL. CANCER INST. 59(4): 1119-1126, 1977

**Description**

This invention relates to the preparation of inhibiting agents against the proliferation of malignant tumour cells derived from animals, including human beings.

As we shall explain hereinbelow, we have found that if malignant tumour cells are cultivated, then removed from the culture medium, an inhibiting agent may be extracted from the culture medium. Moreover, we have found that if the culture takes place at a temperature in the range of 34 to 35.5°C, the extracted inhibiting agent exhibits a remarkably enhanced inhibiting activity as compared with inhibiting agents extracted from cultures at other temperatures. We have found that administration of the inhibiting agent so produced significantly reduces the survival numbers of malignant tumour cells. Side effects of the kind produced by conventional anti-neoplastic agents have not been encountered.

EP-A-87087 discloses a process for the preparation of a physiologically active substance having a molecular weight of 40,000 to 48,000 wherein a culture broth is purified that has been obtained by culturing cells originating from animals or their fused cells in a culture broth at 37°C to which an inducer such as lipopolysaccharides and lipid A's has been added so that the cells may be stimulated.

EP-A-102801 discloses a process for the preparation of an inhibiting agent against the proliferation of malignant tumor cells of human beings therein malignant tumor cells of human beings are cultured at 37°C, and the culture medium is filtered through a 10,000-molecular sieve, a 1,000-molecular sieve, and a 500 molecular sieve to remove the malignant tumor cells.

Biological Abstracts Vol. 66 (1987), n° 41061 discloses that an extract from Harding-Passey mouse melanoma cells contains an active substance that exhibits inhibition of the proliferation of mouse melanoma tumor cells in vitro.

According to a first aspect of the present invention, there is provided a process for preparing an inhibiting agent against the proliferation of malignant tumour cells, characterised in comprising the steps of culturing animal malignant tumour cells at a temperature in the range of 34°C to 35.5°C, and of subjecting the culture medium to ultrafiltration at a molecular weight of 10,000 to 500, thereby removing said malignant tumour cells.

In a second and alternative aspect of the invention, we provide an inhibiting agent against the proliferation of malignant tumour cells, characterised in that said agent is produced by culturing animal malignant tumour cells at a temperature in the range of 34°C to 35.5°C and subjecting the culture medium to ultrafiltration at a molecular weight of 10,000 to 500, thereby removing said malignant tumour cells, and in that said inhibiting agent has a specific activity against the malignant tumour cells which is at least 1.5 times as high as that of an inhibiting agent produced by identical steps except that the temperature of culture is 37°C.

The invention is hereinafter more particularly described by way of practical·examples and with reference to the single figure of the accompanying drawings which illustrate experimental results which we have achieved.

In the experiments hereinafter described, the tumour cells employed were from an established cell line identified HRC originating from a human renal carcinoma. The culture medium employed for extraction was Basal Medium Eagle (hereinafter referred to as BME) for diploid cells supplemented with 10 percent new born calf serum.

Example

An experimental quantity in excess of $2 \times 10^7$ cells from an HRC established cell line were placed with 50 ml of BME in a culture bottle with a base surface area of 150 cm². Samples were allowed to stand at 42°C, 35°C and room temperature (25°C), with a control at 37°C and these respective samples were cultivated at these temperatures for one week. Thereafter, the culture media were filtered through molecular sieves capable of passing substances with the molecular weights of 10,000, 1,000 and 500 respectively and the filtrates were collected.

The following experiments were then performed:

Assay method for the inhibiting effect of the agents against the proliferation of malignant tumour cells:

$10^4$ cells from an established HRC cell line were inoculated in a plastics dish 15 mm in diameter and cultivated in BME with 10% bovine serum for 24 weeks.

To this culture medium and to other essentially identical culture media was added amino acids, vitamins and glucose in the same amounts as in the original BME in the one case and in the other cases additionally inhibiting agents derived as above. The respective cultures were cultivated at 37°C under

conditions of 5% $CO_2$ and 100% humidity for six days. The resultant proliferation of HRC cells in the various cultures was then compared.

Experimental results:

We found that the proliferation of malignant tumour cells in the culture medium containing an inhibiting agent which had been extracted following incubation at around 35°C, as compared with the culture medium containing an inhibiting agent extracted from the culture medium which had been incubated at 37°C, was substantially inhibited.

Table 1 hereinbelow shows the degree of inhibition against proliferation of malignant tumour cells. The figures given were obtained by subtracting the percentage of surviving HRC cells from the original 100%.

### Table 1

| | | Temperatures for incubation | | | | |
|---|---|---|---|---|---|---|
| Experiments | Lot Nos | Room temp | $(25^{O}C)$ | $35^{O}C$ | $37^{O}C$ | $42^{O}C$ |
| 1 | 62-1 | | | | 76% | 46% |
| 2 | 62-2 | | | | 76% | 26% |
| 3 | 63 | | | | 67% | 43% |
| 4 | 65 | | | 21% | 73% | 45% |
| 5 | 66 | | | 25% | 66% | 54% |

It will be seen that the control samples produced by incubation at 37°C produce significant inhibition but that as compared with the inhibition produced by control, the inhibition produced as a result of a sample incubated at 42°C was very much less while again, as compared with the inhibition of the control, the inhibition produced by the sample incubated at 35°C was substantially greater.

In Table 2 set out hereinbelow, the inhibiting activity is expressed as a ratio in each case taking the base as the inhibiting percentage of the control (i.e. 37°C).

### Table 2

| | | Temperatures for incubation | | | | |
|---|---|---|---|---|---|---|
| Experiments | Lot Nos | Room temp | $(25^{O}C)$ | $35^{O}C$ | $37^{O}C$ | $42^{O}C$ |
| 1 | 62-1 | | | | 1 | 0.6 |
| 2 | 62-2 | | | | 1 | 0.3 |
| 3 | 63 | | | 1.6 | 1 | |
| 4 | 65 | | | 0.5 | 1.6 | 1 |
| 5 | 66 | | | 0.5 | 1.2 | 1 |

Referring back to Table 1, it will be seen that the average value of inhibiting rate in the case of the control sample cultivated at 37°C (which is the standard ordinary temperature for incubation) was 58.8%.

We regard inhibiting agents which do not reach an average inhibition value corresponding to this percentage as not being effective.

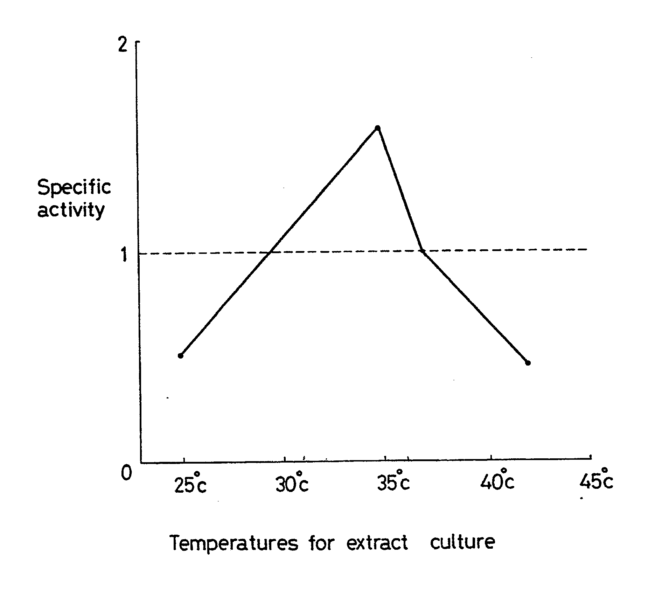

Figure 1 is a graph made from the results in Table 2. The peak in specific activity can clearly be seen at and around 35° C. A specific activity 1.5 times that of the 37° C culture can be inferred from the graph for cultures in the range of 34° C to 35.5° C.

## Claims

1. A process for preparing an inhibiting agent against the proliferation of malignant tumour cells, characterised in comprising the steps of culturing animal malignant tumour cells at a temperature in the range of 34° C to 35.5° C, and of subjecting the culture medium to ultrafiltration at a molecular weight of 10,000 to 500, thereby removing said malignant tumour cells.

2. A process according to claim 1, further characterised in that said animal malignant tumcur cells comprise cells from an established cell line derived from a human renal carcinoma.

3. A process according to Claims 1 or 2, further characterised in that said culture medium comprises Basal Medium Eagle.

4. An inhibiting agent against the proliferation of malignant tumour cells, characterised in that said agent is produced by culturing animal malignant tumour cells at a temperature in the range of 34° C to 35.5° C and subjecting the culture medium to ultrafiltration at a molecular weight of 10,000 to 500, thereby removing said malignant tumour cells, and in that said inhibiting agent has a specific activity against the malignant tumour cells which is at least 1.5 times as high as that of an inhibiting agent produced by identical steps except that the temperature of culture is 37° C.

## Revendications

1. Procédé de préparation d'agents inhibiteurs de la prolifération de cellules de tumeur maligne, caractérisé par le fait qu'il comprend les étapes de mise en culture des cellules d'une tumeur maligne d'animal à une température comprise entre 34° C et 35,5° C, puis à soumettre le milieu de culture à ultrafiltration dans un domaine de poids moléculaires compris entre 10.000 et 500 pour enlever par ce moyen les cellules de la tumeur maligne.

2. Procédé selon la revendication 1, caractérisé en outre par le fait que lesdites cellules de tumeur maligne d'animal comprennent des cellules provenant d'une ligne de cellules fixées, dérivée d'un carcinome de rein humain.

3. Procédé selon l'une ou l'autre des revendications 1 ou 2, caractérisé en outre par le fait que ledit milieu de culture comprend le milieu de base Eagle.

4. Agent inhibiteur de la prolifération des cellules de tumeur maligne, caractérisé en outre par le fait que ledit agent est obtenu à partir d'une mise en culture des cellules d'une tumeur maligne d'animal à une température comprise entre 34° C et 35,5° C puis que l'on soumet le milieu de culture à ultrafiltration dans un domaine de poids moléculaires compris entre 10.000 et 500 pour enlever par ce moyen les cellules de la tumeur maligne et en ce que ledit agent inhibiteur présente une activité spécifique à l'encontre des cellules de tumeur maligne qui est au moins 1,5 fois aussi élevée que celle d'un agent inhibiteur obtenu par des étapes identiques qui ne diffèrent que par le fait que la température de la culture est de 37° C.

## Ansprüche

1. Verfahren zur Herstellung eines Gegenmittels gegen die Proliferation von malignen Tumorzellen, gekennzeichnet durch die Schritte, animalische maligne Tumorzellen bei einer Temperatur im Bereich

EP 0 118 286 B1

von 34°C bis 35,5°C zu züchten und das Kultursubstrat einer Ultrafiltration bei einem Molekulargewicht von 10.000 bis 500 zu unterziehen, wodurch die malignen Tumorzellen beseitigt werden.

2. Verfahren nach Anspruch 1,
dadurch gekennzeichnet, daß die animalischen malignen Tumorzellen Zellen aus einer aufgebauten
Zellverbindung enthalten, die aus einem menschlichen Nieren-Carcinom entnommen worden ist.

3. Verfahren nach den Ansprüchen 1 oder 2,
dadurch gekennzeichnet, daß das Kultursubstrat ein Eagle-Grundmedium enthält.

4. Gegenmittel gegen die Proliferation von malignen Tumorzellen,
dadurch gekennzeichnet, daß das Mittel dadurch hergestellt wird, daß animalische maligne Tumorzellen
bei einer Temperatur im Bereich von 34°C bis 35,5°C gezüchtet werden und das Kultursubstrat einer
Ultrafiltration bei einem Molekulargewicht von 10.000 bis 500 unterzogen wird, wodurch die malignen
Tumorzellen beseitigt werden, und daß das Gegenmittel eine besondere Wirkung gegen maligne
Tumorzellen besitzt, welche mindestens 1,5-fach so hoch wie die eines Gegenmittels ist, das unter
identischen Schritten mit der Ausnahme, daß die Temperatur der Kultur 37°C beträgt, hergestellt
worden ist.

6

Temperatures for extract culture